# EUROPÄISCHE PATENTSCHRIFT

(11) **EP 1 117 661 B1**
(45) Veröffentlichungstag und Bekanntmachung des Hinweises auf die Patenterteilung: **26.05.2004**
(21) Anmeldenummer: 99948790.3
(22) Anmeldetag: 18.09.1999
(51) Int. Cl.: C07D 401/12, C07D 417/12, C07D 401/14, C07D 417/14, C07D 413/14, C07D 409/14, C07D 409/12, A61K 31/4427

(54) **MIT HETEROCYCLEN SUBSTITUIERTE PROPANOLAMINDERIVATE, VERFAHREN ZU DEREN HERSTELLUNG, DIESE VERBINDUNGEN ENTHALTENDE ARZNEIMITTEL UND DEREN VERWENDUNG**
PROPANOLAMINE DERIVATIVES SUBSTITUTED WITH HETEROCYCLIC COMPOUNDS, METHODS FOR THEIR PRODUCTION, PHARMACEUTICAL COMPOSITIONS CONTAINING SAID COMPOUNDS AND THE USE THEREOF
DERIVES DE PROPANOLAMINE SUBSTITUES PAR HETEROCYCLES, PROCEDES PERMETTANT DE LES PREPARER, MEDICAMENTS CONTENANT LESDITS COMPOSES ET LEUR UTILISATION

(30) Priorität: 02.10.1998 DE 19845402
(43) Veröffentlichungstag der Anmeldung: 25.07.2001
(73) Patentinhaber: Aventis Pharma Deutschland GmbH, 65929 Frankfurt am Main (DE)
(72) Erfinder: FRICK, Wendelin, D-65510 Hünstetten-Beuerbach (DE); KIRSCH, Reinhard, D-38100 Braunschweig (DE); GLOMBIK, Heiner, D-65719 Hofheim (DE); HEUER, Hubert, D-55270 Schwabenheim (DE)
(86) Internationale Anmeldenummer: PCT/EP1999/006932
(87) Internationale Veröffentlichungsnummer: WO 2000/020410

(56) Entgegenhaltungen:
- EP-A- 0 869 121
- Y H ET AL: "Hypolipidemic effects of alpha, beta, and gamma-alkylaminophenone analogs in rodents" EUROPEAN JOURNAL OF MEDICINAL CHEMISTRY.CHIMICA THERAPEUTICA,FR,EDITIONS SCIENTIFIQUE ELSEVIER, PARIS, Bd. 31, Nr. 4, Seite 281-290 XP004040088 ISSN: 0223-5234

## Beschreibung

Die Erfindung betrifft substituierte Propanoiaminderivate und deren pharmazeutisch verträgliche Salze.

Es sind bereits mehrere Wirkstoffklassen zur Behandlung von Adipositas und von Lipidstoffwechselstörungen beschrieben worden:
- polymere Adsorber, wie z.B. Cholestyramin
- Benzothiazepine (WO 93/16055)
- Gallensäuredimere und -konjugate (EP 0 489 423)
- 4-Amino-2-ureido-pyrimidin-5-carbonsäureamide (EP 0 557 879)

Der Erfindung lag die Aufgabe zugrunde, weitere Verbindungen zur Verfügung zu stellen, die eine therapeutisch verwertbare hypolipidämische Wirkung entfalten.

Die Erfindung betrifft daher Verbindungen der Formel I, 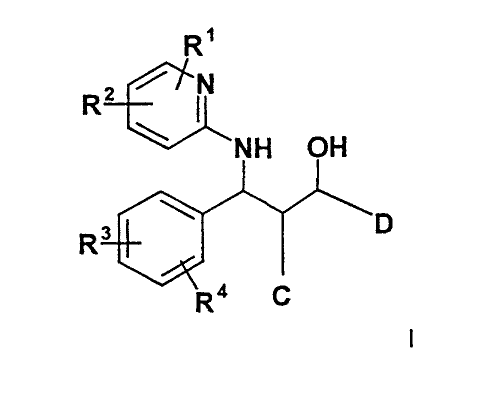 worin bedeuten
- C: Phenyl, Pyridyl, Thienyl, Furyl, Pyrimidyl, Indolyl, Thiazolyl, Imidazolyl, Coumarinyl, Phthalimidyl, Chinolyl, Piperazinyl, Tetrazolyl, Triazolyl, Oxazolyl Isoxazolyl, Isothiazolyl oder deren thieno-, pyridinooder benzoannelierte Derivate, wobei der Aromat oder Heteroaromat ein bis zweifach substituiert sein kann mit Fluor, Chlor, Brom, Jod, OH, CF₃, -NO₂, CN, (C₁-C₈)-Alkoxy, (C₁-C₈)-Alkyl, NH₂, -NH-R⁹, -N(R⁹)R¹⁰, CHO, -COOH, -COOR¹¹, -(C=O)-R¹², (C₁-C₆)-alkyl-OH, (C₁-C₆)-alkyl(-OH)-Phenyl, (C₁-C₆)-alkyl-CF₃, (C₁-C₆)-alkyl-NO₂, (C₁-C₆)-alkyl-CN, (C₁-C₆)-alkyl-NH₂, (C₁-C₆)-alkyl-NH-R⁹, (C₁-C₆)-alkyl-N(R⁹)R¹⁰, (C₁-C₆)-alkyl-CHO, (C₁-C₆)-alkyl-COOH, (C₁-C₆)-alkyl-COOR¹¹, (C₁-C₆)-alkyl-(C=O)-R¹², -O-(C₁-C₆)-alkyl-OH, -O-(C₁-C₆)-alkyl-CF₃, -O-(C₁-C₆)-alkyl-NO₂, -O-(C₁-C₆)-alkyl-CN, -O-(C₁-C₆)-alkyl-NH₂, -O-(C₁-C₆)-alkyl-NH-R⁹, -O-(C₁-C₆)-alkyl-N(R⁹)R¹⁰, -O-(C₁-C₆)-alkyl-CHO, -O-(C₁-C₆)-alkyl-COOH, -O-(C₁-C₆)-alkyl-COOR¹¹, -O-(C₁-C₆)-alkyl-(C=O)-R¹², -N-SO₃H, -SO₂-CH₃, -O-(C₁-C₆)-alkyl-O-(C₁-C₆)-alkyl-Phenyl, wobei in den Alkylresten ein oder mehrere Wasserstoff(e) durch Fluor ersetzt sein können;
- D: Phenyl, Pyridyl, Thienyl, Furyl, Pyrimidyl, Indolyl, Thiazolyl, Imidazolyl, Coumarinyl, Phthalimidyl, Chinolyl, Piperazinyl, Tetrazolyl, Triazolyl, Oxazolyl lsoxazolyl, Isothiazofyl, 4,5,6,7-tetrahydrobenzisoxazol oder deren thieno-, pyridino- oder benzoannelierte Derivate, wobei der Aromat oder Heteroaromat ein bis zweifach substituiert sein kann mit Fluor, Chlor, Brom, Jod, OH, CF₃, -NO₂, CN, (C₁-C₈)-Alkoxy, (C₁-C₈)-Alkyl, NH₂, -NH-R⁹, -N(R⁹)R¹⁰, CHO, -COOH, -COOR¹¹, -(C=O)-R¹², (C₁-C₆)-alkyl-OH, (C₁-C₆)-alkyl(-OH)-Phenyl, (C₁-C₆)-alkyl-CF₃, (C₁-C₆)-alkyl-NO₂, (C₁-C₆)-alkyl-CN, (C₁-C₆)-alkyl-NH₂, (C₁-C₆)-alkyl-NH-R⁹, (C₁-C₆)-alkyl-N(R⁹)R¹⁰, (C₁-C₆)-alkyl-CHO, (C₁-C₆)-alkyl-COOH, (C₁-C₆)-alkyl-COOR¹¹, (C₁-C₆)-alkyl-(C=O)-R¹², -O-(C₁-C₆)-alkyl-OH, -O-(C₁-C₆)-alkyl-CF₃, -O-(C₁-C₆)-alkyl-NO₂, -O-(C₁-C₆)-alkyl-CN, -O-(C₁-C₆)-alkyl-NH₂, -O-(C₁-C₆)-alkyl-NH-R⁹, -O-(C₁-C₆)-alkyl-N(R⁹)R¹⁰, -O-(C₁-C₆)-alkyl-CHO, -O-(C₁-C₆)-alkyl-COOH, -O-(C₁-C₆)-alkyl-COOR¹¹, -O-(C₁-C₆)-alkyl-(C=O)-R¹², -N-SO₃H, -SO₂-CH₃, -C₀-C₆)-alkyl-Pyridyl, -O-(C₁-C₆)-alkyl-O-(C₁-C₆)-alkyl-Phenyl, -(C₀-C₆)-alkyl-Phenyl, wobei die Phenylreste bis zu zweimal mit F, Cl, CF₃, OCF₃, (C₁-C₆)-alkyl oder -O-(C₁-C₆)-alkyl substituiert sein können und wobei in den Alkylresten ein oder mehrere Wasserstoff(e) durch Fluor ersetzt sein können;
mit der Maßgabe, daß C und D nicht gleichzeitig die folgende Bedeutung haben:
C = Phenyl und D = Phenyl, C = Phenyl und D = Pyridyl, C = Pyridyl und D = Phenyl, C = Pyridyl und D = Pyridyl;
- R¹, R², R³, R⁴: unabhängig voneinander Wasserstoff, Fluor, Chlor, Brom, Jod, OH, CF₃, -NO₂, CN, (C₁-C₈)-Alkoxy, (C₁-C₈)-Alkyl, NH₂, -NH-R⁹, -N(R⁹)R¹⁰, CHO, -COOH, -COOR¹¹, -(C=O)-R¹², (C₁-C₆)-alkyl-OH, (C₁-C₆)-alkyl(-OH)-Phenyl, (C₁-C₆)-alkyl-CF₃, (C₁-C₆)-alkyl-NO₂, (C₁-C₆)-alkyl-CN, (C₁-C₆)-alkyl-NH₂, (C₁-C₆)-alkyl-NH-R⁹, (C₁-C₆)-alkyl-N(R⁹)R¹⁰, (C₁-C₆)-alkyl-CHO, (C₁-C₆)-alkyl-COOH, (C₁-C₆)-alkyl-COOR¹¹, (C₁-C₆)-alkyl-(C=O)-R¹², -O-(C₁-C₆)-alkyl-OH, -O-(C₁-C₆)-alkyl-CF₃, -O-(C₁-C₆)-alkyl-NO₂, -O-(C₁-C₆)-alkyl-CN, -O-(C₁-C₆)-alkyl-NH₂, -O-(C₁-C₆)-alkyl-NH-R⁹, -O-(C₁-C₆)-alkyl-N(R⁹)R¹⁰, -O-(C₁-C₆)-alkyl-CHO, -O-(C₁-C₆)-alkyl-COOH, -O-(C₁-C₆)-alkyl-COOR¹¹, -O-(C₁-C₆)-alkyl-(C=O)-R¹², -N-SO₃H, -SO₂-CH₃, -O-(C₁-C₆)-alkyl-O-(C₁-C₆)-alkyl-Phenyl, wobei in den Alkylresten ein oder mehrere Wasserstoff(e) durch Fluor ersetzt sein können;
- R⁹ bis R¹²: unabhängig voneinander Wasserstoff, (C₁-C₈)-Alkyl;
sowie deren pharmazeutisch verträgliche Salze.

Bevorzugt sind Verbindungen der Formel 1, in denen ein oder mehrere Rest(e) die folgende Bedeutung hat bzw. haben:
- C: Phenyl, Pyridyl, Thienyl, Furyl, Pyrimidyl, Indolyl, Thiazolyl, Imidazolyl, Coumarinyl, Phthaliminyl, Chinoyl, Piperazinyl, Tetrazolyl, Triazolyl, Oxazolyl Isoxazolyl, Isothiazolyl oder deren benzoannelierte Derivate, wobei der Aromat oder Heteroaromat ein bis zweifach substituiert sein kann mit Fluor, Chlor, Brom, Jod, OH, CF₃, -NO₂, CN, (C₁-C₈)-Alkoxy, (C₁-C₈)-Alkyl, C₃-C₆-Cycloalkyl, NH₂, CHO, -COOH, OCF₃
- D: Phenyl, Pyridyl, Thienyl, Furyl, Pyrimidyl, Indolyl, Thiazolyl, Imidazolyl, Coumarinyl, Phthaliminyl, Chinoyl, Piperazinyl, Tetrazolyl, Triazolyl, Oxazolyl, Isoxazolyl, Isothiazolyl oder deren benzoannelierte Derivate, wobei der Aromat oder Heteroaromat ein bis zweifach substituiert sein kann mit Fluor, Chlor, Brom, Jod, OH, CF₃, -NO₂, CN, (C₁-C₈)-Alkoxy, (C₁-C₈)-Alkyl, C₃-C₆-Cycloalkyl, NH₂, CHO, -COOH, OCF₃;
mit der Maßgabe, daß C und D nicht gleichzeitig die folgende Bedeutung haben:
C = Phenyl und D = Phenyl, C = Phenyl und D = Pyridyl, C = Pyridyl und D = Phenyl, C = Pyridyl und D = Pyridyl;
- R¹, R², R³, R⁴: unabhängig voneinander Wasserstoff, Fluor, Chlor, Brom, Jod, OH, CF₃, OCF₃, NO₂, CN, (C₁-C₈)-Alkoxy, (C₁-C₈)-Alkyl, C₃-C₆-Cycloalkyl, NH₂, -NH-R⁹, -N(R⁹)R¹⁰, CHO, -COOH, -COOR¹¹, -(C=O)-R¹², wobei in den Alkylresten ein oder mehrere Wasserstoff(e) durch Fluor ersetzt sein können;
- R⁹ bis R¹²: unabhängig voneinander Wasserstoff, (C₁-C₈)-Alkyl;
sowie deren pharmazeutisch verträgliche Salze.

Besonders bevorzugt sind Verbindungen der Formel 1, in denen ein oder mehrere Rest(e) die folgende Bedeutung hat bzw. haben:
- C: Phenyl, Pyridyl, Thienyl, Pyrimidyl, Indolyl, Thiazolyl, Chinoyl, Oxazolyl, Isoxazolyl, wobei der Aromat oder Heteroaromat ein bis zweifach substituiert sein kann mit Fluor, Chlor, Brom, (C₁-C₈)-Alkyl;
- D: Phenyl, Pyridyl, Thienyl, Pyrimidyl, Indolyl, Thiazolyl, Chinoyl, Imidazolyl, Triazolyl, Oxazolyl, Isoxazolyl, wobei der Aromat oder Heteroaromat ein bis zweifach substituiert sein kann mit Fluor, Chlor, Brom, (C₁-C₈)-Alkyl;
mit der Maßgabe, daß C und D nicht gleichzeitig die folgende Bedeutung haben:
C = Phenyl und D = Phenyl, C = Phenyl und D = Pyridyl, C = Pyridyl und D = Phenyl, C = Pyridyl und D = Pyridyl;
- R¹, R², R³, R⁴: unabhängig voneinander Wasserstoff, Fluor, Chlor, Brom, Jod, OH, CF₃, OCF₃, NO₂, CN, (C₁-C₈)-Alkoxy, (C₁-C₈)-Alkyl, C₃-C₆-Cycloalkyl, NH₂, -NH-R⁹, -N(R⁹)R¹⁰, CHO, -COOH, -COOR¹¹, -(C=O)-R¹², wobei in den Alkylresten ein oder mehrere Wasserstoff(e) durch Fluor ersetzt sein können;
- R⁹ bis R¹²: unabhängig voneinander Wasserstoff, (C₁-C₈)-Alkyl;
sowie deren pharmazeutisch verträgliche Salze.

Unter dem Begriff Alkyl werden geradkettige oder verzweigte Kohlenwasserstoffketten verstanden.

Die Erfindung betrifft weiterhin ein Verfahren zur Herstellung von Verbindungen der Formel I, das durch folgendes Reaktionsschema gekennzeichnet ist:

### Verfahren A

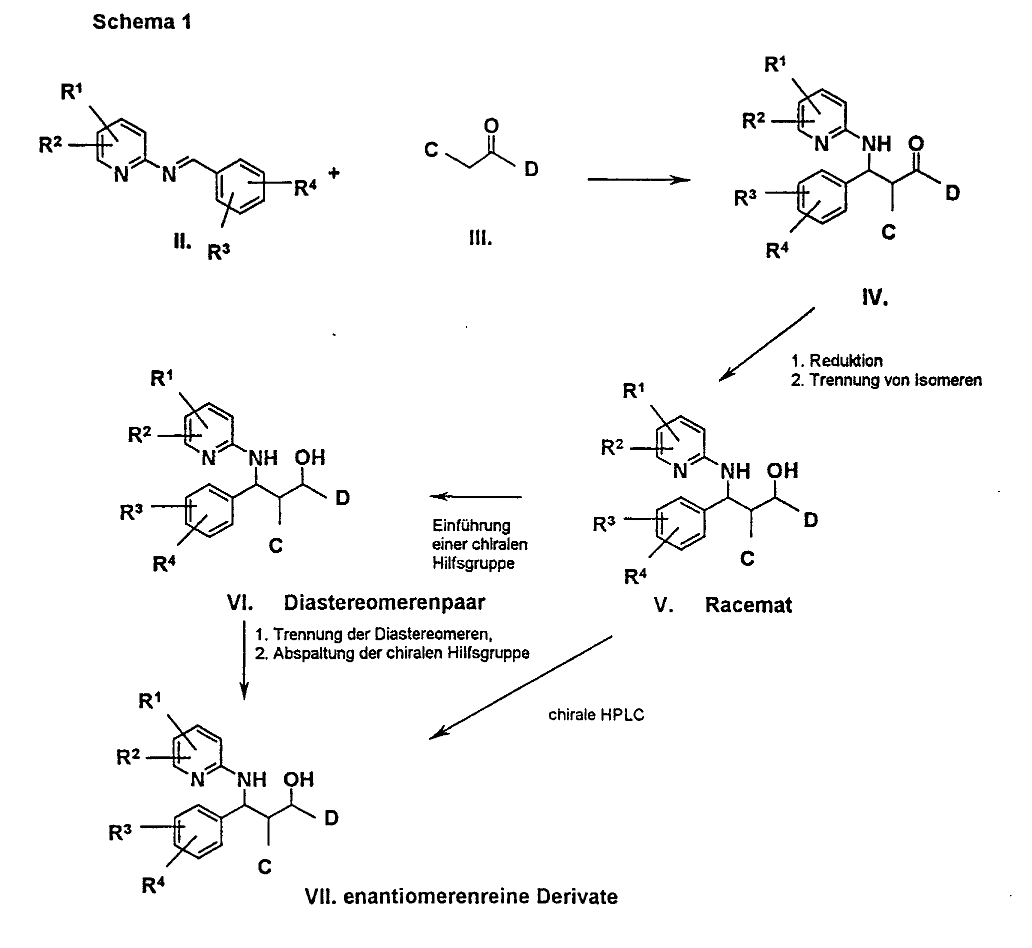

Verbindungen des Typs IV werden erhalten, indem o-, m- oder p-substituierte Imine des Typs II mit dem Keton III zur Reaktion gebracht werden. Die Reaktion kann zum Beispiel durch Mischung der beiden Verbindungen in Substanz, ohne Lösungsmittel, und anschließendem Erhitzen oder in einem geeigneten Lösungsmittel wie Ethanol, Tetrahydrofuran (THF), Toluol, Diglyme oder Tetradecan bei Temperaturen von 20° C bis 150° C durchgeführt werden.

Die Ketoverbindungen des Typs IV werden in einem geeigneten Lösungsmittel, wie z.B. Methanol, THF oder THF/Wasser mit NaBH₄ oder einem anderen geeigneten Reduktionsmittel bei Temperaturen zwischen -30° C und + 40° C zu Hydroxyverbindungen des Typs V reduziert. Bei der Reduktion fallen bis zu vier Isomerengemische (Racemate) als Reaktionsprodukte an. Die unterschiedlichen Racemate können durch fraktionierte Kristallisation oder durch Kieselgelchromatographie voneinander abgetrennt werden.

Die so erhaltenen racemischen Verbindungen des Typs V können weiterhin in ihre Enantiomere aufgetrennt werden. Die Racematspaltung von V in Enantiomere des Typs VII kann durch Chromatographie über chirales Säulenmaterial oder durch literaturbekannte Verfahren mit optisch aktiven Hilfsreagenzien durchgeführt werden (vgl. J. Org. Chem. **44**, 1979, 4891).

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isothion-, Milch-, Lactobion-, Malein-, Apfel-, Methansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natriumund Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (1) wie vorstehend beschrieben, sowie ihre Salze, Solvate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des vom Salz abgeleiteten Benzothiazepin-lons. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen.

Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenfalls gepufferten wäßrigen Lösung, gelöst und/oder dispergiert in einem Haftmittel oder dispergiert in einem Polymer. Eine geeignete Wirkstoff-Konzentration beträgt ca. 1 % bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Erfindung bezieht sich auf Verbindungen der Formel I, in Form ihrer Racemate, racemischen Mischungen und reinen Enantiomere sowie auf ihre Diastereomere und Mischungen davon.

Die Alkyl-, Alkenyl- und Alkinylreste in den Substituenten R1, R1', R2, R3 und R4 können sowohl geradkettig wie verzweigt sein.

Die Verbindungen der Formel I und deren pharmazeutisch verträgliche Salze und physiologisch funktionelle Derivate zeichnen sich durch günstige Wirkungen auf den Lipidstoffwechsel aus. Die Verbindungen können allein oder in Kombination mit weiteren lipidsenkenden Wirkstoffen eingesetzt werden. Die Verbindungen eignen sich zur Prophylaxe sowie insbesondere zur Behandlung von Lipidstoffwechselstörungen, insbesondere von Hyperlipidämie. Die Verbindungen der Formel I eignen sich ebenfalls zur Beeinflussung des Serumcholesterinspiegels sowie zur Prävention und Behandlung arteriosklerotischer Erscheinungen.
Folgende Befunde belegen die pharmakologische Wirksamkeit der erfindungsgemäßen Verbindungen.

Die biologische Prüfung der erfindungsgemäßen Verbindungen erfolgte durch Ermittlung der Hemmung der [³H]-Taurocholataufnahme in Bürstensaummembranvesikel des lleums von Kaninchen. Der Hemmtest wurde wie folgt durchgeführt:

### 1. Präparation von Bürstensaummembranvesikeln aus dem lleum von Kaninchen

Die Präparation von Bürstensaummembranvisikeln aus den Darmzellen des Dünndarm erfolgte mit der sogenannten Mg²⁺-Präzipitationsmethode. Männliche Neuseeland-Kaninchen (2 bis 2,5 kg Körpergewicht) wurden durch intravenöse Injektion von 0,5 ml T61®, einer wäßrigen Lösung von 2,5 mg Tetracain HCl, 100 mg Embutramid und 25 mg Mebezoniumjodid getötet. Der Dünndarm wurde entnommen und mit eiskalter physiologischer Kochsalzlösung gespült. Die terminalen 7/10 des Dünndarms (gemessen in oral-rektaler Richtung, d.h. das terminale lleum, welches das aktive Na⁺-abhängige Gallensäuretransportsystem enthält) wurden zur Präparation der Bürstensaummembranvesikel verwendet. Die Därme wurden in Kunststoffbeuteln unter Stickstoff bei -80°C eingefroren. Zur Präparation der Membranvesikel wurden die eingefrorenen Därme bei 30°C im Wasserbad aufgetaut. Die Mucosa wurde abgeschabt und in 60 ml eiskaltem 12 mM Tris/HCl-Puffer (pH 7,1)/300 mM Mannit, 5 mM EGTA/10 mg/l Phenylmethyl-sulfonylfluorid/1 mg/l Trypsin Inhibitor v. Sojabohnen (32 U/mg)/0,5 mg/l Trypsin Inhibitor v. Rinderlunge (193 U/mg)/5 mg/l Bacitracin suspendiert. Nach dem Verdünnen auf 300 ml mit eiskaltem destilliertem Wasser wurde mit einem Ultraturrax (18-Stab, IKA Werk Staufen, Deutschland) 3 Minuten bei 75 % max. Leistung unter Eiskühlung homogenisiert. Nach Zugabe von 3 ml 1 M MgCl₂-Lösung (Endkonzentration 10 mM) ließ man exakt 1 Minute bei 0□C stehen. Durch Zugabe von Mg²⁺ aggregieren die Zellmembranen und präzipitieren mit Ausnahme der Bürstensaummembranen. Nach einer 15-minütigen Zentrifugation bei 3000 x g (5000 rpm, SS-34-Rotor) wird der Niederschlag verworfen und der Überstand, der die Bürstensaummembranen enthält, 30 Minuten bei 48000 x g (20000 rpm, SS-34-Rotor) zentrifugiert. Der Überstand wurde verworfen, der Niederschlag in 60 ml 12 mM Tris/HCl-Puffer (pH 7,1)/60 mM Mannit, 5 mM EGTA mit einem Potter Elvejhem Homogenisator (Braun, Melsungen, 900 rpm, 10 Hübe) rehomogenisiert. Nach Zugabe von 0,1 ml 1 M MgCl₂-Lösung und 15-minütiger Inkubationszeit bei 0°C wurde erneut 15 Minuten bei 3000 x g zentrifugiert. Der Überstand wurde anschließend nochmals 30 Minuten bei 48000 x g (20000 rpm, SS-34-Rotor) zentrifugiert. Der Niederschlag wurde in 30 ml 10 mM Tris/Hepes-Puffer (pH 7,4)/300 mM Mannit aufgenommen und durch 20 Hübe in einem Potter Elvejhem Homogenisator bei 1000 rpm homogen resuspendiert. Nach 30 minütiger Zentrifugation bei 48000 x g (20000 rpm, SS-34-Rotor) wird der Niederschlag in 0,5 bis 2 ml Tris/Hepes-Puffer (pH 7,4)/280 mM Mannit (Endkonzentration 20 mg/ml) aufgenommen und mit Hilfe einer Tuberkulinspritze mit einer 27 Gauge-Nadel resuspendiert. Die Vesikel wurden entweder unmittelbar nach der Präparation für Transportuntersuchungen verwendet oder bei -196°C in 4 mg Portionen in flüssigem Stickstoff aufbewahrt.

### 2. Hemmung der Na⁺-abhängigen [³H]Taurocholat-Aufnahme in Bürstensaummembranvesikel des lleums

Die Aufnahme von Substraten in die vorstehend beschriebenen Bürstensaummembranvesikel wurde mittels der sogenannten Membranfiltrationstechnik bestimmt. 10 µl der Vesikelsuspension (100 µg Protein) wurden als Tropfen an die Wand eines Polystyrolinkubationsröhrchens (11 x 70 mm) pipettiert, welches das Inkubationsmedium mit den entsprechenden Liganden enthielt (90 µl). Das Inkubationsmedium enthielt 0,75 µl = 0,75 µCi [³H(G)]-Taurocholat (spezifische Aktivität: 2,1 Ci/mMol)/0,5 µl 10 mM Taurocholat/8,75 µl Natrium-Transport-Puffer (10 mM Tris/Hepes (pH 7,4)/100 mM Mannit/1 00 mM NaCl) (Na-T-P) bzw. 8,75 µl Kalium-Transport-Puffer (10 mM Tris/Hepes (pH 7,4)/100 mM Mannit/100 mM KCl) (K-T-P) und 80 µl der betreffenden Inhibitorlösung, je nach Experiment in Na-T-Puffer bzw. K-T-Puffer gelöst. Das Inkubationsmedium wurde durch ein Polyvinylidenfluorid-Membranfilter (SYHV LO 4NS, 0,45 µm, 4 mm ⌀, Millipore, Eschborn, Deutschland) filtriert. Durch Vermischung der Vesikel mit dem Inkubationsmedium wurde die Transportmessung gestartet. Die Konzentration an Taurocholat im Inkubationsansatz betrug 50 µM. Nach der gewünschten Inkubationszeit (üblicherweise 1 Minute) wurde der Transport durch Zugabe von 1 ml eiskalter Stoplösung (10 mM Tris/Hepes (pH 7,4)/150 mM KCI) gestoppt. Die entstehende Mischung wurde sofort bei einem Vakuum von 25 bis 35 mbar über ein Membranfilter aus Cellulosenitrat (ME 25, 0,45 µm, 25 mm Durchmesser, Schleicher & Schuell, Dassell, Deutschland) abgesaugt. Der Filter wurde mit 5 ml eiskalter Stoplösung nachgewaschen.

Zur Messung der Aufnahme des radioaktiv markierten Taurocholats wurde das Membranfilter mit 4 ml des Szintillators Quickszint 361 (Zinsser Analytik GmbH, Frankfurt, Deutschland) aufgelöst und die Radioaktivität durch Flüssigkeits-Szintillationsmessung in einem Meßgerät TriCarb 2500 (Canberra Packard GmbH, Frankfurt, Deutschland) gemessen. Die gemessenen Werte wurden nach Eichung des Gerätes mit Hilfe von Standardproben und nach Korrektur evtl. vorhandener Chemilumineszenz als dpm (Decompositions per Minute) erhalten.

Die Kontrollwerte wurden jeweils in Na-T-P und K-T-P ermittelt. Die Differenz zwischen der Aufnahme in Na-T-P und K-T-P ergab den Na⁺-abhängigen Transportanteil. Als IC₅₀ Na⁺ wurde diejenige Konzentration an Inhibitor bezeichnet, bei der der Na⁺-abhängige Transportanteil um 50 % - bezogen auf die Kontrolle - gehemmt war.
Die pharmakologischen Daten umfassen eine Testserie, in der die Interaktion der erfindungsgemäßen Verbindungen mit dem intestinalen Gallensäuretransportsystem im terminalen Dünndarm untersucht wurde. Die Ergebnisse sind in Tabelle 1 zusammengefaßt.

Tabelle 1 zeigt Meßwerte (Biolog. Test) der Hemmung der [³H]-Taurocholataufnahme in Bürsensaummembranvesikel des lleums von Kaninchen. Angegeben sind die Quotienten aus den IC_{50Na}-Werten der Referenzsubstanz als Taurochenodesoxycholat (TCDC) und der jeweiligen Testsubstanz.

Die nachfolgenden Beispiele dienen zur näheren Erläuterung der Erfindung, ohne dieselbe auf in den Beispielen beschriebene Produkte und Ausführungsformen einzuschränken.

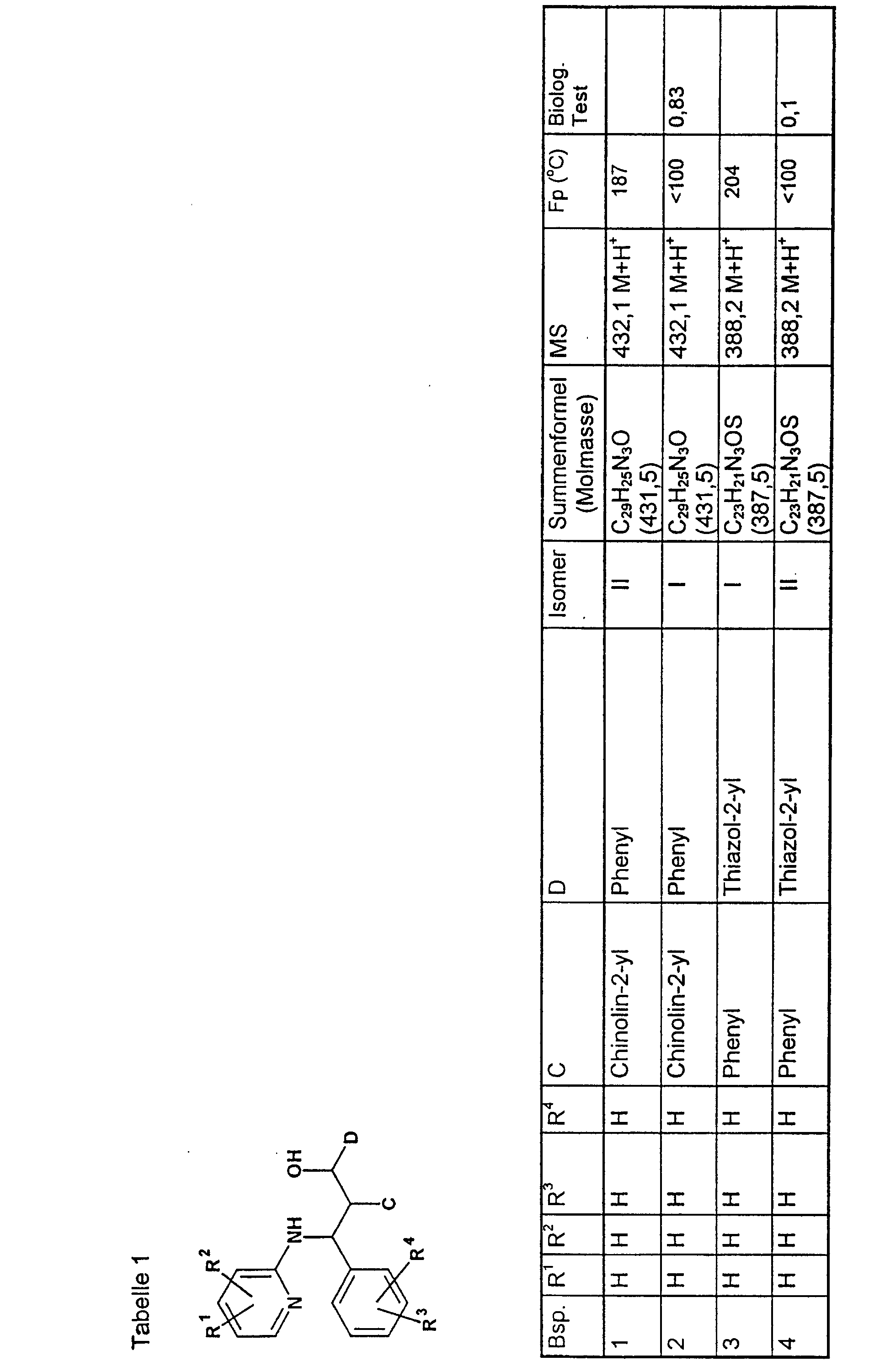

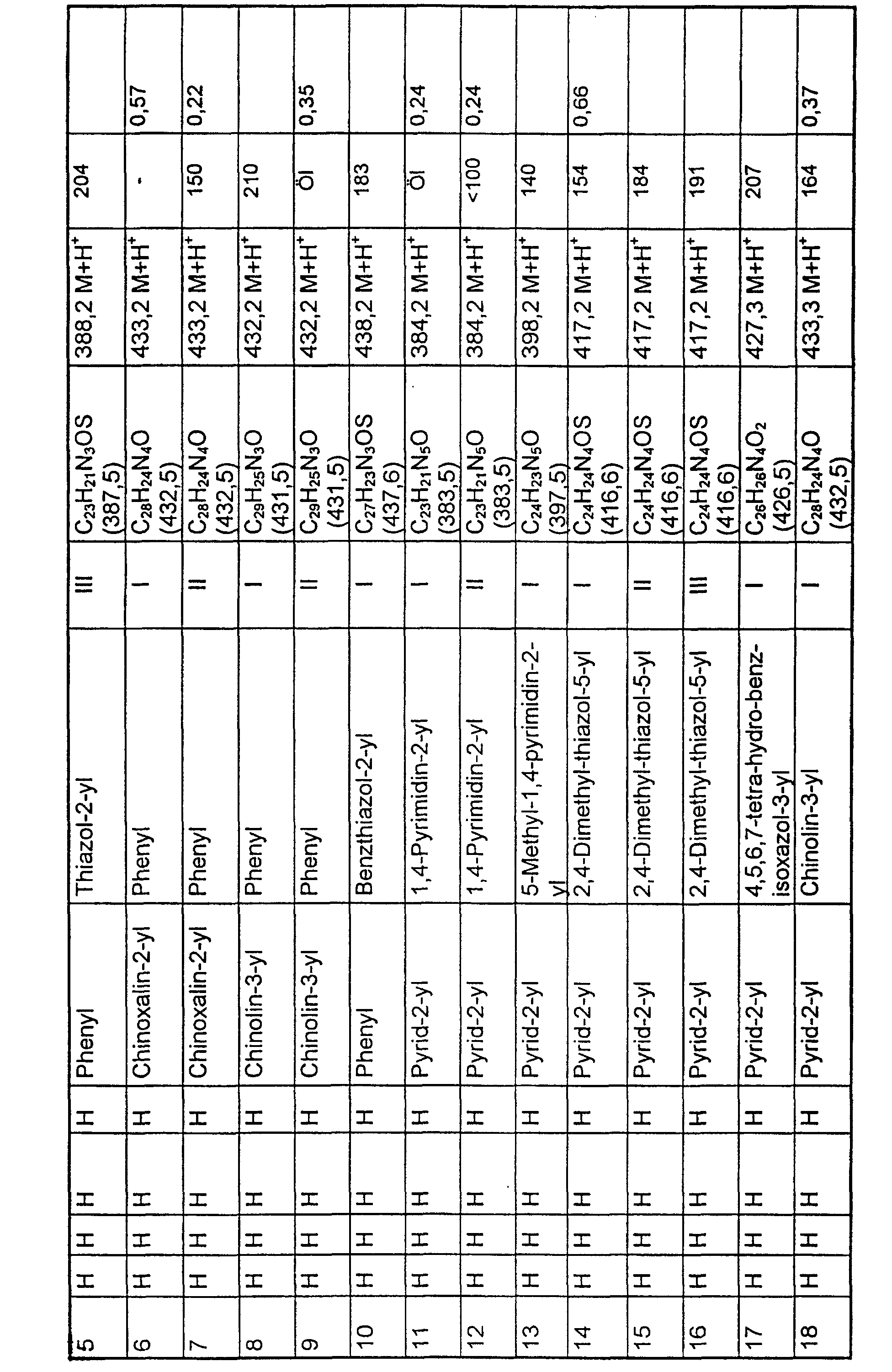

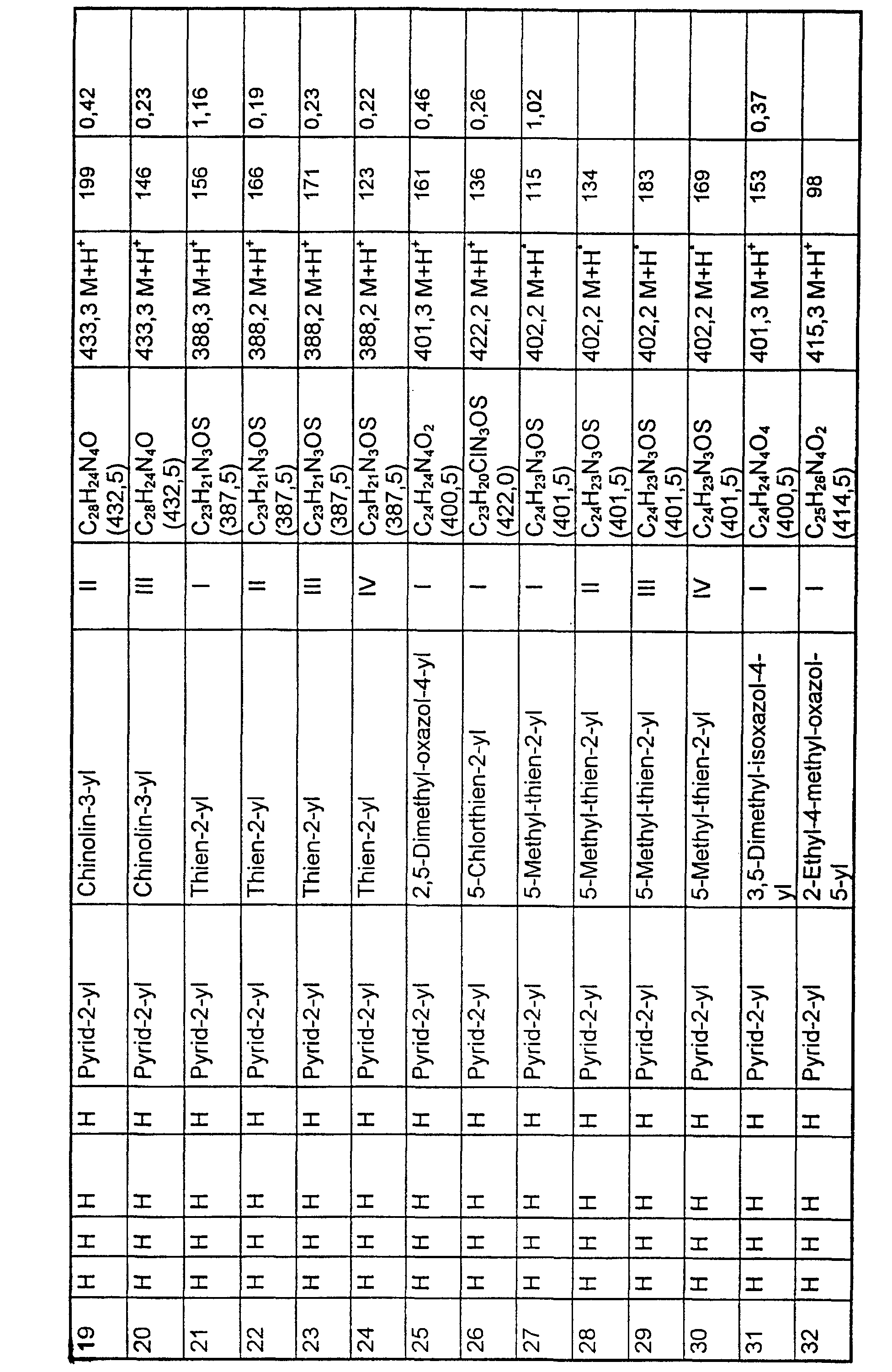

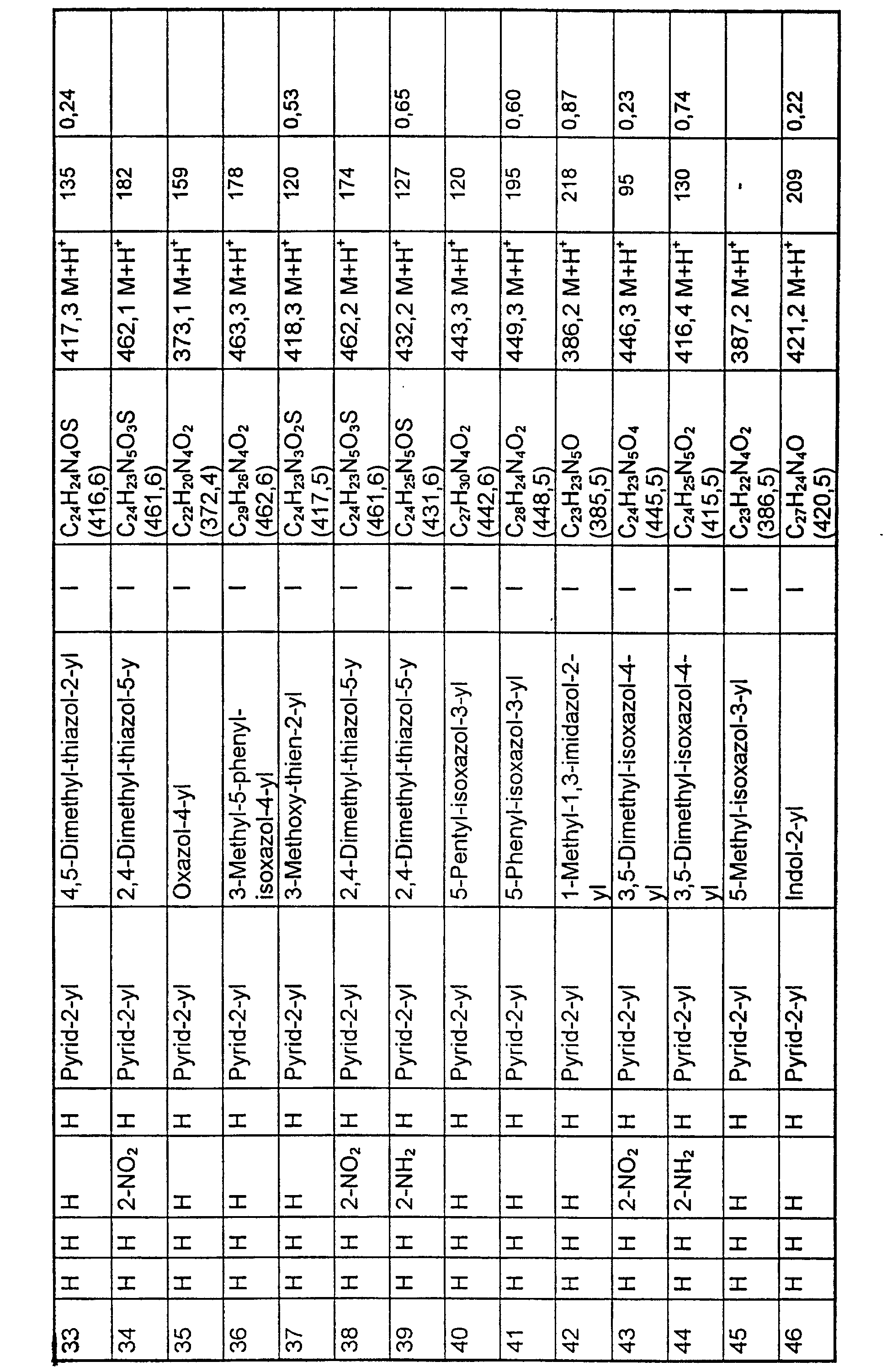

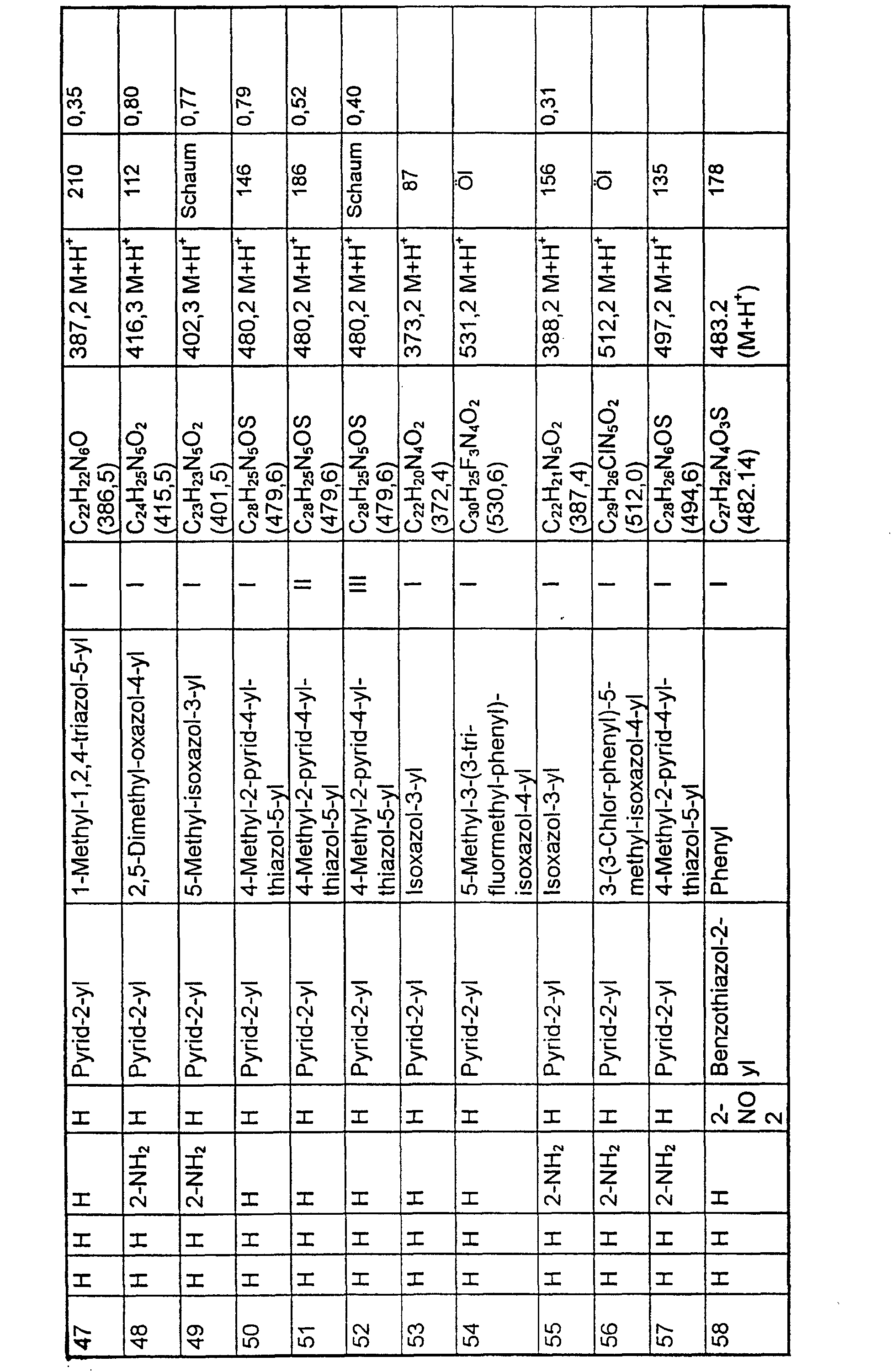

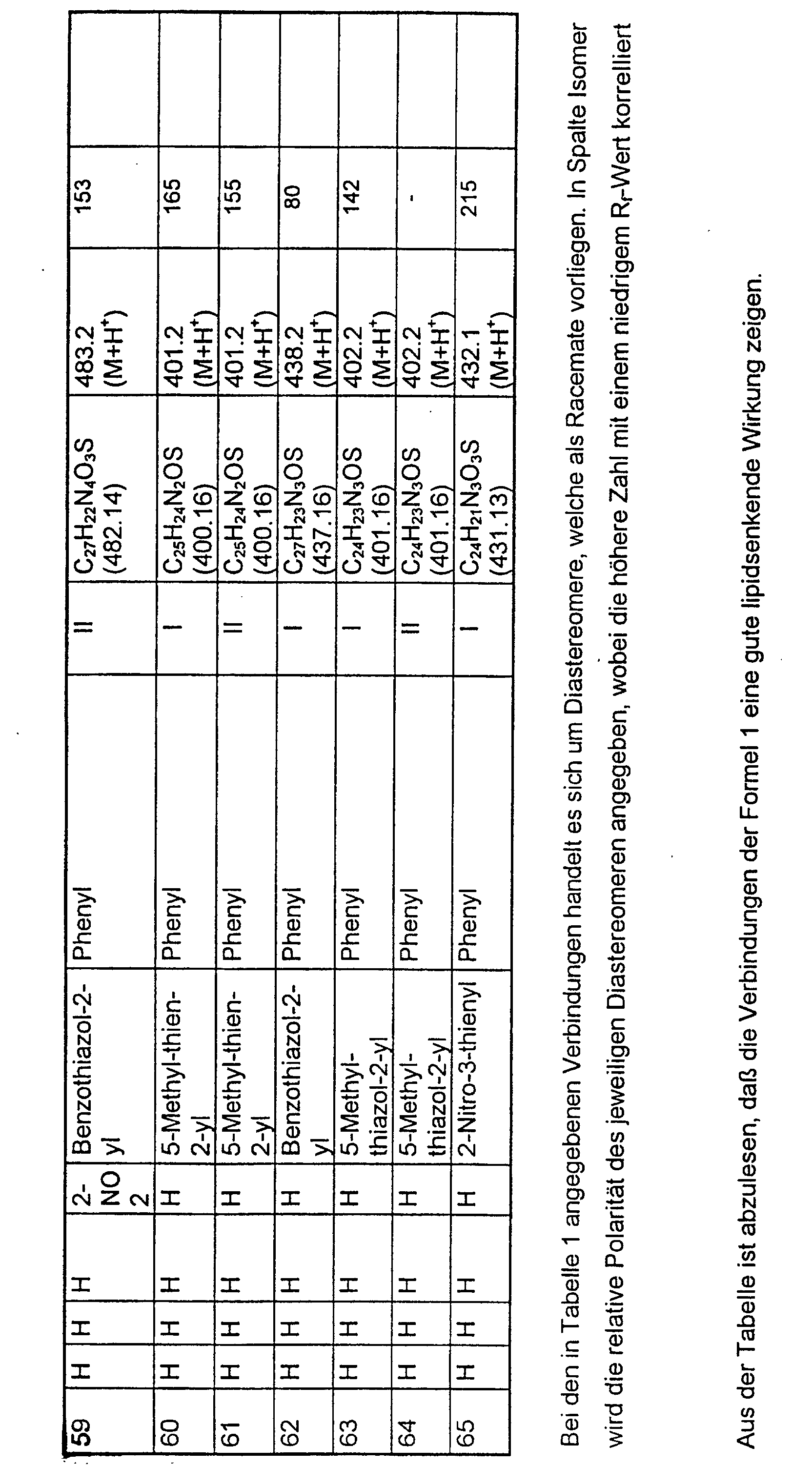

Nachfolgend wird die Herstellung einiger Beispiele detailliert beschrieben, die übrigen Verbindungen der Formel I (siehe Tabelle 1) wurden aus den entsprechenden Ausgangsverbindungen analog erhalten:

### Beispiel A

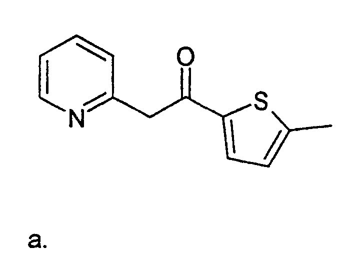

Zu 5,6 g (0.06 mol) Picolin in 50 ml abs. Tetrahydrofuran werden bei -60°C 38 ml 15 % n-Butyllithium in n-Hexan zugetropft. Es wird auf Zimmertemperatur erwärmt und erneut auf -60°C gekühlt. 8,5 g 5-Methylthiophen-2-carbonsäure (0.05 mol) in 15 ml Tetrahydrofuran wird langsam zugetropft, anschließend auf Zimmertemperatur erwärmt und noch 1 h gerührt. Nach der Zugabe von 300 ml Wasser und Neutralisation mittels 20% wässriger Citronensäurelösung wird mit 100 ml Dichlormethan extrahiert (3x), die organische Phasen mit Na₂SO₄ getrocknet und unter reduziertem Druck eingedampft. Nach Chromatographie an Kieselgel mit n-Heptan/Ethylacetat als mobiler Phase erhält man
2,6 g (24 % d. Th)
des Reaktionsproduktes in Form eines hellgelben Öls.
C₁₂H₁₁NOS (217,3) MS 218,2 M+H⁺

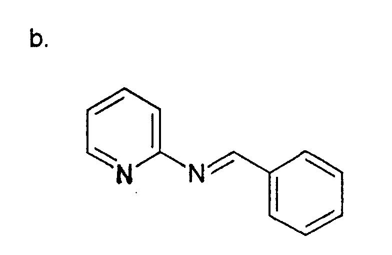

51 ml (0.5 mol) Benzaldehyd, 47 g (0.5 mol) 2-Aminopyridin und 1 g p-Toluolsulfonsäure werden in 400 ml Toluol gelöst und 3 h am Wasserabscheider unter Rückfluß erhitzt. Die Lösung wird abgekühlt, die organische Phase zweimal mit ges. wäßriger NaHCO₃-Lösung und zweimal mit je 100 ml Wasser gewaschen. Anschließend wird mittels Na₂SO₄ getrocknet und unter reduziertem Druck eingeengt. Das als Öl erhaltene Rohprodukt wird im Ölpumpenvakuum destilliert.
- Ausbeute:: 73,8 g (81 % d. Th) Produkt
- Kp_{0,2}:: 125°C
- C₁₂H₁₂N₂ (182,2): MS 183,3 M + H⁺

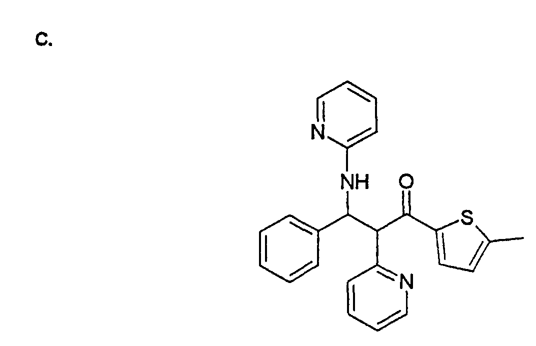

### (Gemisch zweier Diastereomere)

2,6 g (12 mmol) Keton aus Beispiel 1 a und 2,2 g (12 mmol) Imin aus Beispiel 1 b werden in 50 ml Ethanol gelöst. Nach wenigen Minuten beginnt ein farbloser Feststoff auszufallen. Zur Vervollständigung der Reaktion wird 48 h bei Zimmertemperatur gerührt. Nach dem Abkühlen wird der Niederschlag abgesaugt und aus Ethanol umkristallisiert.
- Ausbeute:: 3,45 g (72% d.Th.) Produkt
Schmelzpunkt: 160°C

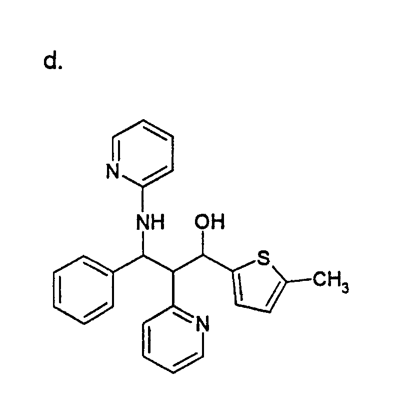

(Herstellung der vier möglichen Diastereomeren, s. Bsp. 27 bis 30 in Tabelle 1) 3,4 g (8,5 mmol) Ketoverbindung aus Beispiel 1 c werden in einer Mischung aus 350 ml Dichlormethan, 25 ml Methanol und 8 ml Wasser gelöst, mit 2,4 g Natriumborhydrid versetzt und 5 h bei Zimmertemperatur gerührt. Anschließend wird die Lösung zweimal mit 150 ml Wasser extrahiert und die organische Phase mit Na₂SO₄ getrocknet und eingedampft. Der Rückstand wird über Kieselgel chromatographiert (n-Heptan/Ethylacetat 1:1). Es werden vier, jeweils racemische, Verbindungen als farblose, kristalline Produkte erhalten:
- 1. Fraktion:: 1,1 g (32 %) stark unpolares Racemat (Bsp. 27);
R_{f}(Ethylacetat/n-Heptan=1/1 ): 0,37
Schmelzpunkt: 115°C
C₂₄H₂₃N₃OS (401,5) MS (FAB) 402,2 M + H⁺
- 2. Fraktion:: 0,32 g (9 %) unpolares Racemat (Bsp. 28)
R_{f}(Ethylacetat/n-Heptan=1/1): 0,30
Schmelzpunkt: 134°C
C₂₄H₂₃N₃OS (401,5) MS (FAB) 402,2 M+H⁺
- 3. Fraktion:: 0,54 g (16 %) mittelpolares Racemat (Bsp. 29)
R_{f}(Ethytacetat/n-Heptan=1/1): 0,22
Schmelzpunkt: 183°C
C₂₄H₂₃N₃OS(401,5) MS (FAB) 402,2 M + H⁺
- 4. Fraktion:: 0,38 g (11 %) polares Racemat (Bsp. 30)
R_{f}(Ethylacetat/n-Heptan=1/1): 0,16
Schmelzpunkt: 169°C
C₂₄H₂₃N₃OS (401,5) MS (FAB) 402,2 M + H⁺

## Patentansprüche

1. Verbindungen der Formel I, 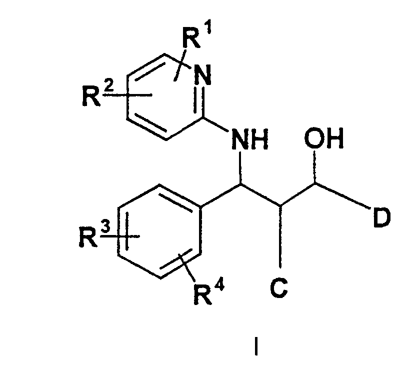 worin bedeuten
C Phenyl, Pyridyl, Thienyl, Furyl, Pyrimidyl, Indolyl, Thiazolyl, Imidazolyl, Coumarinyl, Phthalimidyl, Chinoyl, Piperazinyl, Tetrazolyl, Triazolyl, Oxazolyl Isoxazolyl, Isothiazolyl oder deren thieno-, pyridinooder benzoannelierte Derivate, wobei der Aromat oder Heteroaromat ein bis zweifach substituiert sein kann mit Fluor, Chlor, Brom, Jod, OH, CF₃, -NO₂, CN, (C₁-C₈)-Alkoxy, (C₁-C₈)-Alkyl, NH₂, -NH-R⁹, -N(R⁹)R¹⁰, CHO, -COOH, -COOR¹¹, -(C=O)-R¹², (C₁-C₆)-alkyl-OH, (C₁-G₆)-alkyl(-OH)-Phenyl, (C₁-C₆)-alkyl-CF₃, (C₁-C₆)-alkyl-NO₂, (C₁-C₆)-alkyl-CN, (C₁-C₆)-alkyl-NH₂, (C₁-C₆)-alkyl-NH-R⁹, (C₁-C₆)-alkyl-N(R⁹)R¹⁰, (C₁-C₆)-alkyl-CHO, (C₁-C₆)-alkyl-COOH, (C₁-C₆)-alkyl-COOR¹¹, (C₁-C₆)-alkyl-(C=O)-R¹², -O-(C₁-C₆)-alkyl-OH, -O-(C₁-C₆)-alkyl-CF₃, -O-(C₁-C₆)-alkyl-NO₂, -O-(C₁-C₆)-alkyl-CN, -O-(C₁-C₆)-alkyl-NH₂, -O-(C₁-C₆)-alkyl-NH-R⁹, -O-(C₁-C₆)-alkyl-N(R⁹)R¹⁰, -O-(C₁-C₆)-alkyl-CHO, -O-(C₁-C₆)-alkyl-COOH, -O-(C₁-C₆)-alkyl-COOR¹¹, -O-(C₁-C₆)-alkyl-(C=O)-R¹², -N-SO₃H, -SO₂-CH₃, -O-(C₁-C₆)-alkyl-O-(C₁-C₆)-alkyl-Phenyl, wobei in den Alkylresten ein oder mehrere Wasserstoff(e) durch Fluor ersetzt sein können;
D Phenyl, Pyridyl, Thienyl, Furyl, Pyrimidyl, Indolyl, Thiazolyl, Imidazolyl, Coumarinyl, Phthalimidyl, Chinoyl, Piperazinyl, Tetrazolyl, Triazolyl, Oxazolyl Isoxazolyl, isothiazolyl, 4,5,6,7-tetrahydrobenzisoxazol oder deren thieno-, pyridino- oder benzoannelierte Derivate, wobei der Aromat oder Heteroaromat ein bis zweifach substituiert sein kann mit Fluor, Chlor, Brom, Jod, OH, CF₃, -NO₂, CN, (C₁-C₈)-Alkoxy, (C₁-C₈)-Alkyl, NH₂, -NH-R⁹, -N(R⁹)R¹⁰, CHO, -COOH, -COOR¹¹, -(C=O)-R¹², (C₁-C₆)-alkyl-OH, (C₁-C₆)-alkyl(-OH)-Phenyl, (C₁-C₆)-alkyl-CF₃, (C₁-C₆)-alkyl-NO₂, (C₁-C₆)-alkyl-CN, (C₁-C₆)-alkyl-NH₂, (C₁-C₆)-alkyl-NH-R-⁹, (C₁-C₆)-alkyl-N(R⁹)R¹⁰, (C₁-C₆)-alkyl-CHO, (C₁-C₆)-alkyl-COOH, (C₁-C₆)-alkyl-COOR¹¹, (C₁-C₆)-alkyl-(C=O)-R¹², -O- (C₁-C₆)-alkyl-OH, -O-(C₁-C₆)-alkyl-CF₃, -O-(C₁-C₆)-alkyl-NO₂, -O-(C₁-C₆)-alkyl-CN, -O-(C₁-C₆)-alkyl-NH₂, -O-(C₁-C₆)-alkyl-NH-R⁹, -O-(C₁-C₆)-alkyl-N(R⁹)R¹⁰, -O-(C₁-C₆)-alkyl-CHO, -O-(C₁-C₆)-alkyl-COOH, -O-(C₁-C₆)-alkyl-COOR¹¹, -O-(C₁-C₆)-alkyl-(C=O)-R¹², -N-SO₃H, -SO₂-CH₃, -(C₀-C₆)-alkyl-Pyridyl, -O-(C₁-C₆)-alkyl-O-(C₁-C₆)-alkyl-Phenyl, -(C₀-C₆)-alkyl-Phenyl, wobei die Phenylreste bis zu zweimal mit F, Cl, CF₃, OCF₃, (C₁-C₆)-alkyl oder -O-(C₁-C₆)-alkyl substituiert sein können und wobei in den Alkylresten ein oder mehrere Wasserstoff(e) durch Fluor ersetzt sein können;
mit der Maßgabe, daß C und D nicht gleichzeitig die folgende Bedeutung haben:
C = Phenyl und D = Phenyl, C = Phenyl und D = Pyridyl, C = Pyridyl und D = Phenyl, C = Pyridyl und D = Pyridyl;
R¹, R², R³, R⁴ unabhängig voneinander Wasserstoff, Fluor, Chlor, Brom, Jod, OH, CF₃, -NO₂, CN, (C₁-C₈)-Alkoxy, (C₁-C₈)-Alkyl, NH₂, -NH-R⁹, -N(R⁹)R¹⁰, CHO, -COOH, -COOR¹¹, -(C=O)-R¹², (C₁-C₆)-alkyl-OH, (C₁-C₆)-alkyl(-OH)-Phenyl, (C₁-C₆)-alkyl-CF₃, (C₁-C₆)-alkyl-NO₂, (C₁-C₆)-alkyl-CN, (C₁-C₆)-alkyl-NH₂, (C₁-C₆)-alkyl-NH-R⁹, (C₁-C₆)-alkyl-N(R⁹)R¹⁰, (C₁-C₆)-alkyl-CHO, (C₁-C₆)-alkyl-COOH, (C₁-C₆)-alkyl-COOR¹¹, (C₁-C₆)-alkyl-(C=O)-R¹², -O-(C₁-C₆)-alkyl-OH, -O-(C₁-C₆)-alkyl-CF₃, -O-(C₁-C₆,)-alkyl-NO₂, -O-(C₁-C₆,)-alkyl-CN, -O-(C₁-C₆)-alkyl-NH₂, -O-(C₁-C₆)-alkyl-NH-R⁹, -O-(C₁-C₆)-alkyl-N(R⁹)R¹⁰, -O-(C₁-C₆)-alkyl-CHO, -O-(C₁-C₆)-alkyl-COOH, -O-(C₁-C₆)-alkyl-COOR¹¹, -O-(C₁-C₆)-alkyl-(C=O)-R¹², -N-SO₃H, -SO₂-CH₃, -O-(C₁-C₆)-alkyl-O-(C₁-C₆)-alkyl-Phenyl, wobei in den Alkylresten ein oder mehrere Wasserstoff(e) durch Fluor ersetzt sein können;
R⁹ bis R¹² unabhängig voneinander Wasserstoff, (C₁-C₈)-Alkyl;
sowie deren pharmazeutisch verträgliche Salze.

2. Verbindungen der Formel I, gemäß Anspruch 1, **dadurch gekennzeichnet, daß** darin bedeuten
C Phenyl, Pyridyl, Thienyl, Furyl, Pyrimidyl, Indolyl, Thiazolyl, Imidazolyl, Coumarinyl, Phthaliminyl, Chinolyl, Piperazinyl, Tetrazolyl, Triazolyl, Oxazolyl Isoxazolyl, Isothiazolyl oder deren benzoannelierte Derivate, wobei der Aromat oder Heteroaromat ein bis zweifach substituiert sein kann mit Fluor, Chlor, Brom, Jod, OH, CF₃, -NO₂, CN, (C₁-C₈)-Alkoxy, (C₁-C₈)-Alkyl, C₃-C₆-Cycloalkyl, NH₂, CHO, -COOH, OCF₃
D Phenyl, Pyridyl, Thienyl, Furyl, Pyrimidyl, Indolyl, Thiazolyl, Imidazolyl, Coumarinyl, Phthaliminyl, Chinolyl, Piperazinyl, Tetrazolyl, Triazolyl, Oxazolyl, Isoxazolyl, Isothiazolyl oder deren benzoannelierte Derivate, wobei der Aromat oder Heteroaromat ein bis zweifach substituiert sein kann mit Fluor, Chlor, Brom, Jod, OH, CF₃, -NO₂, CN, (C₁-C₈)-Alkoxy, (C₁-C₈)-Alkyl, C₃-C₆-Cycloalkyl, NH₂, CHO, -COOH, OCF₃;
mit der Maßgabe, daß C und D nicht gleichzeitig die folgende Bedeutung haben:
C = Phenyl und D = Phenyl, C = Phenyl und D = Pyridyl, C = Pyridyl und D = Phenyl, C = Pyridyl und D = Pyridyl;
R¹, R², R³, R⁴ unabhängig voneinander Wasserstoff, Fluor, Chlor, Brom, Jod, OH, CF₃, OCF₃, NO₂, CN, (C₁-C₈)-Alkoxy, (C₁-C₈)-Alkyl, C₃-C₆-Cycloalkyl, NH₂, -NH-R⁹, -N(R⁹)R¹⁰, CHO, -COOH, -COOR¹¹, -(C=O)-R¹², wobei in den Alkylresten ein oder mehrere Wasserstoff(e) durch Fluor ersetzt sein können;
R⁹ bis R¹² unabhängig voneinander Wasserstoff, (C₁-C₈)-Alkyl;
sowie deren pharmazeutisch verträgliche Salze.

3. Verbindungen der Formel I, gemäß Anspruch 1 oder 2, **dadurch gekennzeichnet, daß** darin bedeuten
C Phenyl, Pyridyl, Thienyl, Pyrimidyl, Indolyl, Thiazolyl, Chinoyl, Oxazolyl, Isoxazolyl, wobei der Aromat oder Heteroaromat ein bis zweifach substituiert sein kann mit Fluor, Chlor, Brom, (C₁-C₈)-Alkyl;
D Phenyl, Pyridyl, Thienyl, Pyrimidyl, Indolyl, Thiazolyl, Chinoyl, Imidazolyl, Triazolyl, Oxazolyl, Isoxazolyl, wobei der Aromat oder Heteroaromat ein bis zweifach substituiert sein kann mit Fluor, Chlor, Brom, (C₁-C₈)-Alkyl;
mit der Maßgabe, daß C und D nicht gleichzeitig die folgende Bedeutung haben:
C = Phenyl und D = Phenyl, C = Phenyl und D = Pyridyl, C = Pyridyl und D = Phenyl, C = Pyridyl und D = Pyridyl;
R¹, R², R³, R⁴ unabhängig voneinander Wasserstoff, Fluor, Chlor, Brom, Jod, OH, CF₃, OCF₃, NO₂, CN, (C₁-C₈)-Alkoxy, (C₁-C₈)-Alkyl, C₃-C₆-Cycloalkyl, NH₂, -NH-R⁹, -N(R⁹)R¹⁰, CHO, -COOH, -COOR¹¹, -(C=O)-R¹², wobei in den Alkylresten ein oder mehrere Wasserstoff(e) durch Fluor ersetzt sein können;
R⁹ bis R¹² unabhängig voneinander Wasserstoff, (C₁-C₈)-Alkyl;
sowie deren pharmazeutisch verträgliche Salze.

4. Arzneimittel enthaltend eine oder mehrere der Verbindungen gemäß einem oder mehreren der Ansprüche 1 bis 3.

5. Arzneimittel enthaltend eine oder mehrere der Verbindungen gemäß einem oder mehreren der Ansprüche 1 bis 3 und ein oder mehrere lipidsenkende Wirkstoffe.

6. Verbindungen gemäß einem oder mehreren der Ansprüche 1 bis 3 zur Anwendung als Medikament zur Prophylaxe oder Behandlung von Lipidstoffwechselstörungen.

7. Verbindungen gemäß einem oder mehreren der Ansprüche 1 bis 3 zur Anwendung als Medikament zur Behandlung von Hyperlipidämie.

8. Verbindungen gemäß einem oder mehreren der Ansprüche 1 bis 3 zur Anwendung als Medikament zur Prophylaxe oder Behandlung von arteriosklerotischer Erscheinungen.

9. Verbindungen gemäß einem oder mehreren der Ansprüche 1 bis 3 in Kombination mit mindestens einem weiteren lipidsenkenden Wirkstoff zur Anwendung als Medikament zur Prophylaxe oder Behandlung von Lipidstoffwechselstörungen.

10. Verbindungen gemäß einem oder mehreren der Ansprüche 1 bis 3 in Kombination mit mindestens einem weiteren lipidsenkenden Wirkstoff als Medikament zur Behandlung von Hyperlipidämie.

11. Verbindungen gemäß einem oder mehreren der Ansprüche 1 bis 3 in Kombination mit mindestens einem weiteren lipidsenkenden Wirkstoff zur Anwendung als Medikament zur Prophylaxe oder Behandlung von arteriosklerotischer Erscheinungen.

12. Verfahren zur Herstellung eines Arzneimittels enthaltend eine oder mehrere der Verbindungen gemäß einem oder mehreren der Ansprüche 1 bis 3, **dadurch gekennzeichnet, daß** der Wirkstoff mit einem pharmazeutisch geeigneten Träger vermischt wird und diese Mischung in eine für die Verabreichung geeignete Form gebracht wird.

13. Verwendung der Verbindungen gemäß einem oder mehreren der Ansprüche 1 bis 3 zur Herstellung eines Medikaments zur Prophylaxe oder Behandlung von Lipidstoffwechsetstörungen.

14. Verwendung der Verbindungen gemäß einem oder mehreren der Ansprüche 1 bis 3 zur Herstellung eines Medikaments zur Behandlung von Hyperlipidämie.

## Claims

1. A compound of the formula I, 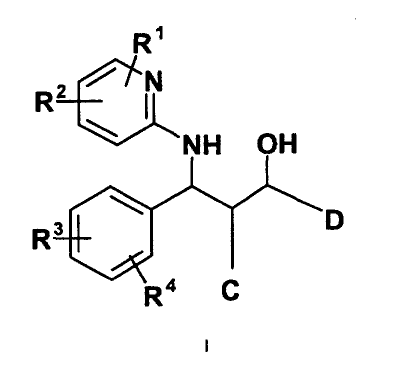 in which
C is phenyl, pyridyl, thienyl, furyl, pyrimidyl, indolyl, thiazolyl, imidazolyl, coumarinyl, phthalimidyl, quinolyl, piperazinyl, tetrazolyl, triazolyl, oxazolyl, isoxazolyl, isothiazolyl or thieno-, pyridino- or benzo-fused derivatives thereof, it being possible for the aromatic or heteroaromatic radical to be mono- to disubstituted by fluorine, chlorine, bromine, iodine, OH, CF₃, -NO₂, CN, (C₁-C₈)-alkoxy, (C₁-C₈)-alkyl, NH₂, -NH-R⁹, - N(R⁹)R¹⁰, CHO, -COOH, -COOR¹¹, -(C=O)-R¹², (C₁-C₆)-alkyl-OH, (C₁-C₆)-alkyl(-OH)-phenyl, (C₁-C₆)-alkyl-CF₃, (C₁-C₆)-alkyl-NO₂, (C₁-C₆)-alkyl-CN, (C₁-C₆)-alkyl-NH₂, (C₁-C₆)-alkyl-NH-R⁹, (C₁-C₆)-alkyl-N(R⁹)R¹⁰, (C₁-C₆)-alkyl-CHO, (C₁-C₆)-alkyl-COOH, (C₁-C₆)-alkyl-COOR¹¹, (C₁-C₆)-alkyl-(C=O)-R¹², -O-(C₁-C₆)-alkyl-OH, -O-(C₁-C₆)-alkyl-CF₃, -O-(C₁-C₆)-alkyl-NO₂, -O-(C₁-C₆)-alkyl-CN, -O-(C₁-C₆)-alkyl-NH₂, -O-(C₁-C₆)-alkyl-NH-R⁹, -O-(C₁-C₆)-alkyl-N(R⁹)R¹⁰, - O-(C₁-C₆)-alkyl-CHO, -O-(C₁-C₆)-alkyl-COOH, -O-(C₁-C₆)-alkyl-COOR¹¹, -O-(C₁-C₆)-alkyl-(C=O)-R¹², -N-SO₃H, -SO₂-CH₃ or -O-(C₁-C₆)-alkyl-O-(C₁-C₆)-alkylphenyl, wherein one or more hydrogen(s) in the alkyl radicals can be replaced by fluorine;
D is phenyl, pyridyl, thienyl, furyl, pyrimidyl, indolyl, thiazolyl, imidazolyl, coumarinyl, phthalimidyl, quinolyl, piperazinyl, tetrazolyl, triazolyl, oxazolyl, isoxazolyl, isothiazolyl or 4,5,6,7-tetrahydrobenzisoxazole or thieno, pyridino- or benzo-fused derivatives thereof, it being possible for the aromatic or heteroaromatic radical to be mono- to disubstituted by fluorine, chlorine, bromine, iodine, OH, CF₃, -NO₂, CN, (C₁-C₈)-alkoxy, (C₁-C₈)-alkyl, NH₂, -NH-R⁹, -N(R⁹)R¹⁰, CHO, -COOH, -COOR¹¹, -(C=O)-R¹², (C₁-C₆)-alkyl-OH, (C₁-C₆)-alkyl(-OH)-phenyl, (C₁-C₆)-alkyl-CF₃, (C₁-C₆)-alkyl-NO₂, (C₁-C₆)-alkyl-CN, (C₁-C₆)-alkyl-NH₂, (C₁-C₆)-alkyl-NH-R⁹, (C₁-C₆)-alkyl-N(R⁹)R¹⁰, (C₁-C₆)-alkyl-CHO, (C₁-C₆)-alkyl-COOH, (C₁-C₆)-alkyl-COOR¹¹, (C₁-C₆)-alkyl-(C=O)-R¹², -O-(C₁-C₆)-alkyl-OH, -O-(C₁-C₆)-alkyl-CF₃, -O-(C₁-C₆)-alkyl-NO₂, -O-(C₁-C₆)-alkyl-CN, -O-(C₁-C₆)-alkyl-NH₂, -O-(C₁-C₆)-alkyl-NH-R⁹, -O-(C₁-C₆)-alkyl-N(R⁹)R¹⁰, -O-(C₁-C₆)-alkyl-CHO, -O-(C₁-C₆)-alkyl-COOH, -O-(C₁-C₆)-alkyl-COOR¹¹, -O-(C₁-C₆)-alkyl-(C=O)-R¹² , -N-SO₃H, -SO₂-CH₃, -(C₀-C₆)-alkyl-pyridyl, -O-(C₁-C₆)-alkyl-O-(C₁-C₆)-alkylphenyl or -(C₀-C₆)-alkylphenyl, wherein the phenyl radicals can be substituted up to twice by F, Cl, CF₃, OCF₃, (C₁-C₆)-alkyl or -O-(C₁-C₆)-alkyl and wherein one or more hydrogen(s) in the alkyl radicals can be replaced by fluorine;
with the proviso that C and D do not simultaneously have the following meaning:
C = phenyl and D = phenyl, C = phenyl and D = pyridyl, C = pyridyl and D = phenyl, C = pyridyl and D = pyridyl;
R¹, R², R³, R⁴ independently of one another are hydrogen, fluorine, chlorine, bromine, iodine, OH, CF₃, -NO₂, CN, (C₁-C₈)-alkoxy, (C₁-C₈)-alkyl, NH₂, -NH-R⁹, -N(R⁹)R¹⁰, CHO, - COOH, -COOR¹¹, -(C=O)-R¹², (C₁-C₆)-alkyl-OH, (C₁-C₆)-alkyl(-OH)-phenyl, (C₁-C₆)-alkyl-CF₃, (C₁-C₆)-alkyl-NO₂, (C₁-C₆)-alkyl-CN, (C₁-C₆)-alkyl-NH₂, (C₁-C₆)-alkyl-NH-R⁹, (C₁-C₆)-alkyl-N(R⁹)R¹⁰, (C₁-C₆)-alkyl-CHO, (C₁-C₆)-alkyl-COOH, (C₁-C₆)-alkyl-COOR¹¹, (C₁-C₆)-alkyl-(C=O)-R¹², -O-(C₁-C₆)-alkyl-OH, -O-(C₁-C₆)-alkyl-CF₃, -O-(C₁-C₆)-alkyl-NO₂, -O-(C₁-C₆)-alkyl-CN, -O-(C₁-C₆)-alkyl-NH₂, -O-(C₁-C₆)-alkyl-NH-R⁹, -O-(C₁-C₆)-alkyl-N(R⁹)R¹⁰, -O-(C₁-C₆)-alkyl-CHO, -O-(C₁-C₆)-alkyl-COOH, -O-(C₁-C₆)-alkyl-COOR¹¹, -O-(C₁-C₆)-alkyl-(C=O)-R¹², -N-SO₃H, -SO₂-CH₃ or -O-(C₁-C₆)-alkyl-O-(C₁-C₆)-alkylphenyl, wherein one or more hydrogen(s) in the alkyl radicals can be replaced by fluorine;
R⁹ to R¹² independently of one another are hydrogen or (C₁-C₈)-alkyl;
and pharmaceutically tolerated salts thereof.

2. A compound of the formula I as claimed in claim 1, in which
C is phenyl, pyridyl, thienyl, furyl, pyrimidyl, indolyl, thiazolyl, imidazolyl, coumarinyl, phthalimidyl, quinolyl, piperazinyl, tetrazolyl, triazolyl, oxazolyl, isoxazolyl, isothiazolyl or benzo-fused derivatives thereof, it being possible for the aromatic or heteroaromatic radical to be mono- to disubstituted by fluorine, chlorine, bromine, iodine, OH, CF₃, -NO₂, CN, (C₁-C₈)-alkoxy, (C₁-C₈)-alkyl, C₃-C₆-cycloalkyl, NH₂, CHO, -COOH or OCF₃
D is phenyl, pyridyl, thienyl, furyl, pyrimidyl, indolyl, thiazolyl, imidazolyl, coumarinyl, phthalimidyl, quinolyl, piperazinyl, tetrazolyl, triazolyl, oxazolyl, isoxazolyl, isothiazolyl or benzo-fused derivatives thereof, it being possible for the aromatic or heteroaromatic radical to be mono- to disubstituted by fluorine, chlorine, bromine, iodine, OH, CF₃, -NO₂, CN, (C₁-C₈)-alkoxy, (C₁-C₈)-alkyl, C₃-C₆-cycloalkyl, NH₂, CHO, -COOH or OCF₃;
with the proviso that C and D do not simultaneously have the following meaning:
C = phenyl and D = phenyl, C = phenyl and D = pyridyl, C = pyridyl and D = phenyl, C = pyridyl and D = pyridyl;
R¹, R², R³, R⁴ independently of one another are hydrogen, fluorine, chlorine, bromine, iodine, OH, CF₃, OCF₃, NO₂, CN, (C₁-C₈)-alkoxy, (C₁-C₈)-alkyl, C₃-C₆-cycloalkyl, NH₂, -NH-R⁹, -N(R⁹)R¹⁰, CHO, -COOH, -COOR¹¹ or -(C=O)-R¹², wherein one or more hydrogen(s) in the alkyl radicals can be replaced by fluorine;
R⁹ to R¹² independently of one another are hydrogen or (C₁-C₈)-alkyl;
and pharmaceutically tolerated salts thereof.

3. A compound of the formula I as claimed in claim 1 or 2, in which
C is phenyl, pyridyl, thienyl, pyrimidyl, indolyl, thiazolyl, quinolyl, oxazolyl, isoxazolyl, it being possible for the aromatic or heteroaromatic radical to be mono- to disubstituted by fluorine, chlorine, bromine or (C₁-C₈)-alkyl;
D is phenyl, pyridyl, thienyl, pyrimidyl, indolyl, thiazolyl, quinolyl, imidazolyl, triazolyl, oxazolyl, isoxazolyl, it being possible for the aromatic or heteroaromatic radical to be mono- to disubstituted by fluorine, chlorine, bromine or (C₁-C₈)-alkyl;
with the proviso that C and D do not simultaneously have the following meaning:
C = phenyl and D = phenyl, C = phenyl and D = pyridyl, C = pyridyl and D = phenyl, C = pyridyl and D = pyridyl;
R¹, R², R³, R⁴ independently of one another are hydrogen, fluorine, chlorine, bromine, iodine, OH, CF₃, OCF₃, NO₂, CN, (C₁-C₈)-alkoxy, (C₁-C₈)-alkyl, C₃-C₆-cycloalkyl, NH₂, -NH-R⁹, -N(R⁹)R¹⁰, CHO, -COOH, -COOR¹¹, or -(C=O)-R¹², wherein one or more hydrogen(s) in the alkyl radicals can be replaced by fluorine;
R⁹ to R¹² independently of one another are hydrogen or (C₁-C₈)-alkyl;
and pharmaceutically tolerated salts thereof.

4. A pharmaceutical comprising one or more of the compounds as claimed in one or more of claims 1 to 3.

5. A pharmaceutical comprising one or more of the compounds as claimed in one or more of claims 1 to 3 and one or more lipid-lowering active compounds.

6. A compound as claimed in one or more of claims 1 to 3 for use as a medicament for the prophylaxis or treatment of disturbances in lipid metabolism.

7. A compound as claimed in one or more of claims 1 to 3 for use as a medicament for the treatment of hyperlipidemia.

8. A compound as claimed in one or more of claims 1 to 3 for use as a medicament for the prophylaxis or treatment of arteriosclerotic symptoms.

9. A compound as claimed in one or more of claims 1 to 3 in combination with at least one further lipid-lowering active compound for use as a medicament for the prophylaxis or treatment of disturbances in lipid metabolism.

10. A compound as claimed in one or more of claims 1 to 3 in combination with at least one further lipid-lowering active compound as a medicament for treatment of hyperlipidemia.

11. A compound as claimed in one or more of claims 1 to 3 in combination with at least one further lipid-lowering active compound for use as a medicament for the prophylaxis or treatment of arteriosclerotic symptoms.

12. A process for the preparation of a pharmaceutical comprising one or more of the compounds as claimed in one or more of claims 1 to 3, which comprises mixing the active compound with a pharmaceutically suitable excipient and bringing this mixture into a form suitable for administration.

13. The use of a compound as claimed in one or more of claims 1 to 3 for the preparation of a medicament for the prophylaxis or treatment of disturbances in lipid metabolism.

14. The use of a compound as claimed in one or more of claims 1 to 3 for the preparation of a medicament for treatment of hyperlipidemia.

## Revendications

1. Composés de la formule I : 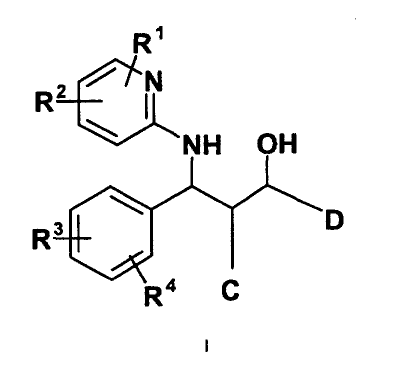 où :
C signifie phényle, pyridyle, thiényle, furyle, pyrimidyle, indolyle, thiazolyle, imidazolyle, coumarinyle, phtalimidyle, quinoléinyle, pipérazinyle, tétrazolyle, triazolyle, oxazolyle, isoxazolyle, isothiazolyle ou leurs dérivés condensés thiéno, pyridino ou benzo, où l'aromatique ou hétéroaromatique peut être substitué une à deux fois avec fluor, chlore, brome, iode, OH, CF₃, NO₂, CN, alcoxy (C₁-C₈) , alkyle (C₁-C₈), NH₂, NH-R⁹, N(R⁹)R¹⁰, CHO, COOH, COOR¹¹, C(O)R¹², alkyl (C₁-C₆) -OH, alkyl (C₁-C₆) (OH) -phényle, alkyl (C₁-C₆) -CF₃, alkyl (C₁-C₆)-NO₂, alkyl (C₁-C₆)-CN, alkyl (C₁-(C₆) -NH₂, alkyl (C₁-C₆) -NH-R⁹, alkyl (C₁-C₆)-N(R⁹)R¹⁰, alkyl (C₁-C₆) -CHO, alkyl (C₁-C₆)-COOH, alkyl (C₁-C₆)-COOR¹¹, alkyl (C₁-C₆)-C(O)R¹², O-alkyl (C₁-C₆)-OH, -O-alkyl (C₁-C₆) -CF₃, -O-alkyl (C₁-C₆)-NO₁, -O-alkyl (C₁-C₆) -CN, -O-alkyl (C₁-C₆) -NH₂, -O-alkyl (C₁-C₆) -NH-R⁹, -O-alkyl (C₁-C₆)-N(R⁹)R¹⁰, -O-alkyl (C₁-C₆)-CHO, -O-alkyl (C₁-C₆)-COOH, -O-alkyl. (C₁-C₆) -COOR¹¹, -O-alkyl (C₁-C₆) -C (O) R¹², -N-SO₃H, -SO₂-CH₃ -O-alkyl (C₁-C₆) -O-alkyl (C₁-C₆) -phényle, où dans les restes alkyle, un ou plusieurs atomes d'hydrogène peuvent être remplaces par fluor ;
D signifie phényle, pyridyle, thiényle, furyle, pyrimidyle, indolyle, thiazolyle, imidazolyle, coumarinyle, phtalimidyle, quinoléinyle, pipérazinyle, tétrazolyle, triazolyle, oxazolyle, isoxazolyle, isothiazolyle, 4,5,6,7-tétrahydrobenzoisoxazole ou leurs dérivés condensés thiéno, pyridino ou benzo, où l'aromatique ou hétéroaromatique peut être substitué une à deux fois avec fluor, chlore, brome, iode, OH, CF₃, NO₂, CN, alcoxy (C₁-C₈), alkyle (C₁-C₈), NH₂, NH-R⁹, N(R⁹)R¹⁰, CHO, COOH, COOR¹¹, C(O)R¹², alkyl (C₁-C₆)-OH, alkyl (C₁-C₆) (OH)-phényle, alkyl (C₁-C₆)-CF₃, alkyl (C₁-C₆)-NO₂, alkyl (C₁-C₆)-CN, alkyl (C₁-C₆)-NH₂, alkyl (C₁-C₆)-NH-R⁹, alkyl (C₁-C₆)-N(R⁹)R¹⁰, alkyl (C₁-C₆)-CHO, alkyl (C₁-C₆)-COOH, alkyl (C₁-C₆) -COOR¹¹, alkyl (C₁-C₆)-C(O)R¹², -O-alkyl (C₁-C₆)-OH, -O-alkyl (C₁-C₆)-CF₃, -O-alkyl (C₁-C₆)-NO₂, -O-alkyl (C₁-C₆)-CN, -O-alkyl (C₁-C₆) -NH₂, -O-alkyl (C₁-C₆)-NH-R⁹, -O-alkyl (C₁-C₆)-N(R⁹)R¹⁰, -O-alkyl (C₁-C₆)-CHO, -O-alkyl (C₁-C₆)-COOH, -O-alkyl (C₁-C₆) -COOR¹¹, -O-alkyl (C₁-C₆)-C(O)R¹², -N-SO₃H, -SO₂-CH₃, -O-alkyl (C₁-C₆)-O-alkyl (C₁-C₆)-phényle, alkyl (C₀-C₆)-phényle, où les restes phényle peuvent être substitués jusqu'à deux fois par F, Cl, CF₃, OCF₃, alkyle (C₁-C₆), -O-alkyle (C₁-C₆), et où dans les restes alkyle, un ou plusieurs atomes d'hydrogène peuvent être remplacés par fluor ;
avec la condition que C et D n'ont pas simultanément, la signification suivante :
C = phényle et D = phényle ; C = phényle et D = pyridyle ; C = pyridyle et D = phényle ; C = pyridyle et D = pyridyle ;
R¹, R², R³, R⁴ représentent indépendamment l'un de l'autre, hydrogène, fluor, chlore, brome, iode, OH, CF₃, NO₂, CN, alcoxy (C₁-C₈), alkyle (C₁-C₈), NH₂, NH-R⁹, N(R⁹)R¹⁰, CHO, COOH, COOR¹¹, C(O)R¹², alkyl (C₁-C₆)-OH, alkyl (C₁-C₆) (OH) -phényle, alkyl (C₁-C₆) -CF₃, alkyl (C₁-C₆) -NO₂, alkyl (C₁-C₆)-CN, alkyl (C₁-C₆)-NH₂, alkyl (C₁-C₆)-NH-R⁹, alkyl (C₁-C₆)-N (R⁹) R¹⁰, alkyl (C₁-C₆)-CHO, alkyl (C₁-C₆)-COOH, alkyl (C₁-C₆)-COOR¹¹, alkyl (C₁-C₆)-C(O)R¹², -O-alkyl (C₁-C₆)-OH, -O-alkyl (C₁-C₆) -CF₃, -O-alkyl (C₁-C₆)-NO₂, -O-alkyl (C₁-C₆) -CN, -O-alkyl (C₁-C₆) -NH₂, -O-alkyl (C₁-C₆) -NH-R⁹, -O-alkyl (C₁-C₆) -N(R⁹)R¹⁰, -O-alkyl (C₁-C₆) -CHO, -O-alkyl (C₁-C₆) -COOH, -O-alkyl (C₁-C₆) -COOR¹¹, -O-alkyl (C₁-C₆)-C(O)R¹², -N-SO₃H, -SO₂-CH₃, -O-alkyl (C₁-C₆) -O-alkyl (C₁-C₆) -phényle, où dans les restes alkyle, un ou plusieurs atomes d'hydrogène peuvent, être remplacés par fluor ;
R⁹ à R¹² représentent indépendamment l'un de l'autre, hydrogène, alkyle (C₁-C₈) ;
ainsi que leurs sels physiologiquement compatibles.

2. Composés de la formule I selon la revendication 1, **caractérisés en ce que** :
C signifie phényle, pyridyle, thiényle, furyle, pyrimidyle, indolyle, thiazolyle, imidazolyle, coumarinyle, phtalimidyle, quinoléinyle, pipérazinyle, tétrazolyle, triazolyle, oxazolyle, isoxazolyle, isothiazolyle ou leurs dérivés condensés benzo, où l'aromatique ou hétéroaromatique peut être substitué une à deux fois avec fluor, chlore, brome, iode, OH, CF₃, NO₂, CN, alcoxy (C₁-C₈), alkyle (C₁-C₈), cycloalkyle (C₃-C₆), NH₂, CHO, COOH, OCF₃ ;
D signifie phényle, pyridyle, thiényle, furyle, pyrimidyle, indolyle, thiazolyle, imidazolyle, coumarinyle, phtalimidyle, quinoléinyle, pipérazinyle, tétrazolyle, triazolyle, oxazolyle, isoxazolyle, isothiazolyle ou leurs dérivés condensés benzo, où l'aromatique ou hétéroaromatique peut être substitué une à deux fois avec fluor, chlore, brome, iode, OH, CF₃, NO₂, CN, alcoxy (C₁-C₈) , alkyle (C₁-C₈), cycloalkyle (C₃-C₆), NH₂, CHO, COOH, OCF₃ ;
avec la condition que C et D n'ont pas simultanément, la signification suivante :
C = phényle et D = phényle ; C = phényle et D = pyridyle ; C = pyridyle et D = phényle ; C = pyridyle et D = pyridyle ;
R¹, R², R³, R⁴ représentent indépendamment l'un de l'autre, hydrogène, fluor, chlore, brome, iode, OH, CF₃, OCF₃, NO₂, CN, alcoxy (C₁-C₈), alkyle (C₁-C₈), cycloalkyle (C₃-C₆), NH₂, NH-R⁹, N(R⁹)R¹⁰, CHO, COOH, COOR¹¹, C(O)R¹², où dans les restes alkyle, un ou plusieurs atomes d'hydrogène peuvent être remplacés par fluor ;
R⁹ à R¹² représentent indépendamment l'un de l'autre, hydrogène, alkyle (C₁-C₆) ; ainsi que leurs sels physiologiquement compatibles.

3. Composés de la formule I selon la revendication 1 où 2, **caractérisés en ce que** :
C signifie phényle, pyridyle, thiényle, pyrimidyle, indolyle, thiazolyle, quinoléinyle, oxazolyle, isoxazolyle, où l'aromatique ou hétéroaromatique peut être substitué une à deux fois avec fluor, chlore, brome, alkyle (C₁-C₈) ;
D signifie phényle, pyridyle, thiényle, pyrimidyle, indolyle, thiazolyle, quinoléinyle, imidazolyle, triazolyle, oxazolyle, isoxazolyle, où l'aromatique ou hétéroaromatique peut être substitué une à deux fois avec fluor, chlore, brome, alkyle (C₁-C₈) ;
avec la condition que C et D n'ont pas simultanément, la signification suivante :
C = phényle et D = phényle ; C = phényle et D = pyridyle ; C = pyridyle et D = phényle ; C = pyridyle et D = pyridyle ;
R¹, R², R³, R⁴ représentent indépendamment l'un de l'autre, hydrogène, fluor, chlore, brome, iode, OH, CF₃, OCF₃, NO₂, CN, alcoxy (C₁-C₈) alkyle (C₁-C₈), cycloalkyle (C₃-C₆), NH₂, NH-R⁹, N(R⁹) R¹⁰, CHO, COOH, COOR¹¹, C(O)R¹², où dans les restes alkyle, un ou plusieurs atomes d'hydrogène peuvent être remplacés par fluor ;
R⁹ à R¹² représentent indépendamment l'un de l'autre, hydrogène, alkyle (C₁-C₈) ;
ainsi que leurs sels physiologiquement compatibles.

4. Agent pharmaceutique contenant un ou plusieurs des composés selon une ou plusieurs des revendications 1 à 3.

5. Agent pharmaceutique contenant un ou plusieurs des composés selon une ou plusieurs des revendications 1 à 3 et un ou plusieurs agents actifs hypolipidémiants.

6. Composés selon une ou plusieurs des revendications 1 à 3, à utiliser comme médicament pour la prophylaxie ou le traitement des troubles du métabolisme des lipides.

7. Composés selon une ou plusieurs des revendications 1 à 3, à utiliser comme médicament pour le traitement de l'hyperlipidémie.

8. Composés selon une ou plusieurs des revendications 1 à 3, à utiliser comme médicament pour la prophylaxie ou le traitement de maladies artériosclérotiques.

9. Composés selon une ou plusieurs des revendications 1 à 3, en combinaison avec au moins un autre agent actif hypolipidémiant, à utiliser comme médicament pour la prophylaxie ou le traitement des troubles du métabolisme des lipides.

10. Composés selon une ou plusieurs des revendications 1 à 3, en combinaison avec au moins un autre agent actif hypolipidémiant, à utiliser comme médicament pour le traitement de l'hyperlipidémie.

11. Composés selon une ou plusieurs des revendications 1 à 3, en combinaison avec au moins un autre agent actif hypolipidémiant, à utiliser comme médicament pour la prophylaxie ou le traitement de maladies artériosclérotiques.

12. Procédé de préparation d'un agent pharmaceutique contenant un ou plusieurs composés selon une ou plusieurs des revendications 1 à 3, **caractérisé en ce que** l'agent actif est mélangé à un support pharmaceutique approprié et ce mélange est mis sous une forme appropriée pour l'administration.

13. Utilisation des composés selon une ou plusieurs des revendications 1 à 3, pour préparer un médicament pour la prophylaxie ou le traitement des troubles du métabolisme des lipides.

14. Utilisation des composés selon une ou plusieurs des revendications 1 à 3, pour préparer un médicament pour le traitement de l'hyperlipidémie.
